# EUROPÄISCHE PATENTSCHRIFT

(11) **EP 2 925 842 B1**
(45) Veröffentlichungstag und Bekanntmachung des Hinweises auf die Patenterteilung: **21.02.2018**
(21) Anmeldenummer: 13788767.5
(22) Anmeldetag: 08.11.2013
(51) Int. Cl.: C11B 9/00, A61K 8/37, A61Q 13/00, C11D 3/50

(54) **DUFTSTOFFMISCHUNGEN ENTHALTEND HEXYLSALICYLAT**
AROMATIC SUBSTANCE MIXTURES CONTAINING HEXYL SALICYLATE
MÉLANGES DE PARFUMS CONTENANT DU SALICYLATE D'HEXYLE

(30) Priorität: 27.11.2012 DE 102012221619
(43) Veröffentlichungstag der Anmeldung: 07.10.2015
(73) Patentinhaber: Henkel AG & Co. KGaA, 40589 Düsseldorf (DE)
(72) Erfinder: MEINE, Georg, 40822 Mettmann (DE); BUNN, Ralf, 40223 Düsseldorf (DE); SMYREK, Hubert, 47804 Krefeld (DE); EUTEBACH, Andrea, 42109 Wuppertal (DE); SONNENSCHEIN, Frank, 42781 Haan (DE)
(86) Internationale Anmeldenummer: PCT/EP2013/073369
(87) Internationale Veröffentlichungsnummer: WO 2014/082834

(56) Entgegenhaltungen:
- EP-A1- 1 921 130
- WO-A1-85/03517
- WO-A1-2010/052636
- DE-A1- 19 743 718
- US-A- 4 383 943
- US-A1- 2009 257 974

## Beschreibung

Die Erfindung betrifft Duftstoffmischungen umfassend Salicylat, Mittel, welche entsprechende Duftstoffmischungen enthalten, die Verwendung Salicylat-haltiger Duftstoffmischungen und Mittel zur Verminderung von Schlechtgerüchen sowie ein Verfahren zur Herstellung eines Schlechtgerüche vermindernden Wasch- oder Reinigungsmittels, Weichspülers, Hygienespülers oder Trocknertuchs.

Selbst nach einem gründlichen Wasch- oder Reinigungsvorgang verbleiben auf einer festen oder textilen Oberfläche oder auch auf der menschlichen Haut Keime in einer geringen, gesundheitlich unkritischen und sensorisch über den Geruchsinn nicht wahrnehmbaren Anzahl. Erhalten diese Keime jedoch bei ausreichender Temperatur und Feuchtigkeit Gelegenheit zur Vermehrung, steigt die Anzahl der Keime auf der Oberfläche innerhalb kürzester Zeit so weit an, dass die Keime beziehungsweise deren Umsetzungsprodukte geruchlich wahrnehmbar werden. Diese bei hoher Keimkonzentration auftretenden Gerüche stuft der Verbraucher durchweg als unangenehm ein. Die Gerüche sollen deshalb im Folgenden als Schlechtgerüche bezeichnet werden. Schlechtgerüche treten beispielsweise auf, wenn gewaschene feuchte Wäsche nicht zügig getrocknet wird, z.B. weil sie nach erfolgtem Waschgang nicht aus der Waschmaschine entnommen wurde.

Mittel zur Verminderung von Schlechtgerüchen sind im Stand der Technik bekannt. So enthalten beispielsweise so genannte Hygienespüler, die anstatt oder zusammen mit einem Weichspüler nach dem Waschen von Textilien angewendet werden können, keimabtötende kationische Tenside, welche die Anzahl von Keimen und auf diese Weise Schlechtgerüche vermindern. Die biologische Abbaubarkeit dieser kationischen Tenside wird jedoch diskutiert.

Eine alternative Methode zur Abtötung von Keimen stellt der Einsatz von Silbersalzen dar. Wasserlösliche Silbersalze können beispielsweise in Wasch- oder Reinigungsmitteln eingesetzt werden und haben aufgrund ihrer keimtötenden Wirkung Einfluss auf die Menge an Keimen und damit das Ausmaß an Schlechtgeruch von mit dem Wasch- oder Reinigungsmittel behandelten Oberflächen. Die Silbersalze müssen jedoch in Wasch- oder Reinigungsmitteln in stabilisierter Form eingesetzt werden, damit sie nicht schon während der Herstellung und Lagerung des Wasch- oder Reinigungsmittels mit anderen Wasch- oder Reinigungsmittelbestandteilen reagieren und damit unwirksam werden. So offenbart beispielsweise die deutsche Offenlegungsschrift DE 10 2007 025 561 A1 Wasch- oder Reinigungsmittel mit einer keimtötenden Zusammensetzung aus einem wasserlöslichen Silbersalz und einem Komplexbildner.

Ein anderer Versuch, einen Geruchseindruck zu verbessern, besteht darin, enthaltene Schlechtgerüche nicht zu entfernen, sondern sie nur vor dem Verbraucher zu verbergen. Dies wird erreicht, indem die Schlechtgerüche von Gerüchen überdeckt werden, die der Verbraucher als angenehm einstuft. Nachteil ist hier, dass die Schlechtgerüche noch immer vorhanden sind und nach dem Verfliegen des überdeckenden und als angenehm wahrgenommenen Duftstoffes wieder deutlicher hervortreten können. Zudem ist der Einsatz von Duftstoffen in uneingeschränkter Menge weder kommerziell sinnvoll noch gemäß Detergentienverordnung möglich.

Es besteht folglich weiterhin ein Bedarf an neuen Mitteln zur Verminderung und/oder Verhinderung von Schlechtgerüchen. Dieser Bedarf besteht nicht nur auf dem Gebiet der Wasch- und Reinigungsmittel, sondern auch auf dem Gebiet der Kosmetik und Körperhygiene.

Zur Deckung dieses Bedarfs wird nun eine Duftstoffmischung bereitgestellt, welche Hexylsalicylat und mindestens ein weiteres Salicylat umfasst, wobei das weitere Salicylat kein 3,7,11-Trimethyl-2,6,10-dodecatrien-1-yl-2-hydroxybenzoat ist und wobei die Mischung zusätzlich zum Hexylsalicylat und zum mindestens einen weiteren Slicylat ein drittes Salicylat, das 3,7,11-Trimethyl-2,6,10-dodecatrien-1-yl-2-hydroxybenzoat ist, enthält.

Gegenstand dieser Anmeldung ist eine Duftstoffmischung enthaltend Hexylsalicylat und zusätzlich mindestens ein weiteres Salicylat, wobei das weitere Salicylat kein 3,7,11-Trimethyl-2,6,10-dodecatrien-1-yl-2-hydroxybenzoat ist und wobei die Mischung zusätzlich zum Hexylsalicylat und zum mindestens einen weiteren Slicylat ein drittes Salicylat, das 3,7,11-Trimethyl-2,6,10-dodecatrien-1-yl-2-hydroxybenzoat ist, enthält.

Unter einer Duftstoffmischung im Rahmen der vorliegenden Anmeldung wird eine Mischung enthaltend mindestens drei Duftstoffverbindungen verstanden. Beispiele für Duftstoffverbindungen sind synthetische Produkte vom Typ der Ester, Ether, Aldehyde, Ketone, Alkohole und Kohlenwasserstoffe. Bevorzugt werden Mischungen verschiedener Duftstoffe verwendet, die gemeinsam eine ansprechende Duftnote erzeugen. Solche Parfümöle können auch natürliche Duftstoffgemische enthalten, wie sie aus pflanzlichen Quellen zugänglich sind. Erfindungsgemäße Duftstoffmischungen enthalten Hexylsalicylat und mindestens ein weiteres Salicylat sowie optional weitere Duftstoffverbindungen.

Überraschend wurde gefunden, dass durch den Einsatz von Duftstoffmischungen, welche Hexylsalicylat sowie mindestens ein weiteres Salicylat enthalten, welches nicht 3,7,11-Trimethyl-2,6,10-dodecatrien-1-yl-2-hydroxybenzoat ist und wobei die Mischung zusätzlich zum Hexylsalicylat und zum mindestens einen weiteren Slicylat ein drittes Salicylat, das 3,7,11-Trimethyl-2,6,10-dodecatrien-1-yl-2-hydroxybenzoat ist, enthält, sowohl ein angenehmer Dufteindruck entsteht als auch eine Verminderung von Schlechtgerüchen erzielt werden kann. Hexylsalicylat, 3,7,11-Trimethyl-2,6,10-dodecatrien-1-yl-2-hydroxybenzoat und das mindestens eine weitere Salicylat lassen sich zudem problemlos in bestehende Parfümierungen einarbeiten.

Das mindestens eine weitere Salicylat ist vorzugsweise ausgewählt aus der Gruppe, die gebildet wird durch Cyclohexylsalicylat, Hexenylsalicylat, Methylsalicylat, Benzylsalicylat, Methylsalicylat, n-Amylsalicylat, *i*-Amylsalicylat, Ethylhexylsalicylat, Cyclooctylsalicylat, 2,2,4-(2,4,4-) Trimethylcyclopentylsalicylat als Isomerengemisch, 4-Isopropylcyclohexylsalicylat, Cyclohexylmethylsalicylat, Cycloheptylsalicylat, Cyclooctyl-salicylat und Cyclooct-4-enyl-salicylat.

Besonders bevorzugt wird als weiteres Salicylat *i*-Amylsalicylat, Cyclohexylsalicylat, Benzylsalicylat oder Methylsalicylat eingesetzt. Beim Einsatz dieser Verbindungen als "weiteres Salicylat" wurden sehr gute Ergebnisse im Hinblick auf die Verringerung von Schlechtgerüchen gefunden.

Bevorzugt sind auch erfindungsgemäße Mischungen, die SALICYNILE, CAS-Nr. 130786-09-3 und/oder Hexenylsalicylate cis-3 CAS-Nr. 65405-77-8 enthalten.

Bevorzugt enthält die Duftstoffmischung neben dem Hexylsalicylat und dem einem weiteren Salicylat ein drittes Salicylat. Dieses kann beispielsweise ausgewählt sein aus den oben genannten Salicylaten oder auch 3,7,11-Trimethyl-2,6,10-dodecatrien-1-yl-2-hydroxybenzoat sein. 3,7,11-Trimethyl-2,6,10-dodecatrien-1-yl-2-hydroxybenzoat (Farnesylsalicylat) ist ein Ester aus Salicylsäure (2-Hydroxybenzoesäure) und Farnesol (3,7,11-Trimethyl-2,6,10-dodecatrien-1-ol) und mittels Verersterungsreaktion aus diesen Komponenten darstellbar.

Besonders bevorzugt ist es demnach, wenn die Duftstoffmischung zusätzlich zum Hexylsalicylat und zum mindestens einen weiteren Salicylat auch noch 3,7,11-Trimethyl-2,6,10-dodecatrien-1-yl-2-hydroxybenzoat enthält. Bei dieser Kombination konnten beste Ergebnisse der Schlechtgeruchverminderung bei gleichzeitiger Parfümierung beobachtet werden.

Auch der Einsatz von Hexylsalicylat und drei, vier, fünf oder sechs weiteren Salicylaten ist möglich.

Einen erheblichen Einfluss auf die ausgelobte Wirksamkeit der Duftstoffmischungen hat auch die Menge des enthaltenen Hexylsalicylates. Die Duftstoffmischung enthält deswegen vorzugsweise mindestens 2 Gew.-% Hexylsalicylat und mit Vorzug mindestens 0,5 Gew.-%, insbesondere mindestens 1 Gew.-% an weiteren Salicylaten (Summe aller weiteren Salicylate) - jeweils bezogen auf die gesamte Duftstoffmischung.

In einer bevorzugten Ausführungsform enthält die Duftstoffmischung 5 bis 100 Gew.-% Salicylate, insbesondere 10 bis 30 Gew.-% Salicylate - bezogen auf die Duftstoffmischung.

Bevorzugt wird die Parfümhaftung der restlichen Parfüminhaltsstoffe durch die Mischung der Salicylate verbessert.

Gegenstand dieser Anmeldung sind zudem Mittel umfassend mindestens ein Tensid aus der Gruppe der nichtionischen oder anionischen Tenside und eine Duftstoffmischung enthaltend Hexylsalicylat und zusätzlich mindestens ein weiteres Salicylat, wobei das weitere Salicylat kein 3,7,11-Trimethyl-2,6,10-dodecatrien-1-yl-2-hydroxybenzoat ist. Es wurde gefunden, dass erfindungsgemäße Duftstoffmischungen die beschriebene Wirksamkeit besonders in Kombination mit anionischen und/oder nichtionischen Tensiden aufweist. Dementsprechend enthalten erfindungsgemäße Mittel mindestens ein Tensid ausgewählt aus der Gruppe der anionischen und nichtionischen Tenside.

Es wurde überraschend gefunden, dass Mittel, welche Hexylsalicylat und zusätzlich mindestens ein weiteres Salicylat enthalten, wobei das weitere Salicylat kein 3,7,11-Trimethyl-2,6,10-dodecatrien-1-yl-2-hydroxybenzoat ist, die Schlechtgeruch-vermindernde sowie die parfümierende Wirkung nicht nur direkt nach dem Einsatz des Mittels, sondern über eine längere Zeit aufweisen. Ohne auf eine Theorie festgelegt zu werden, wäre eine Erklärung dieses Beobachtung dadurch möglich, dass es in den Mitteln zu einer partiellen, möglicherweise kontinuierlich über die Zeit erfolgenden Spaltung der Salicylate kommt und die resultierenden über einen längeren Zeitraum freigesetzten Spaltprodukte Schlechtgeruch vermindernde Eigenschaften und/oder einen angenehmen Duft aufweisen.

Vorzugsweise ist das erfindungsgemäße Mittel ein festes Wasch- oder Reinigungsmittel, ein flüssiges Wasch- oder Reinigungsmittel, ein Hygienespüler, ein Aftershaveprodukt, ein Seifenprodukt, ein Duschgel, eine Duschcreme, ein Badesalz, ein Badeschaum, ein Haarpflegeprodukt, ein Shampoo, ein Körperpflegeprodukt, ein Deodorant, eine kosmetische Zubereitung, ein Trocknertuch, ein Bügelwasser oder ein Wischtuch. Alle genannten Mittel ermöglichen neben der Reinigung oder Pflege der jeweiligen Oberfläche zudem die Verhinderung beziehungsweise Verminderung von Schlechtgerüchen und vermitteln dem Verbraucher somit einen angenehmen Geruch der behandelten Oberfläche.

Wie bereits oben beschrieben, stellt sich das Problem der Bildung oder Intensivierung von Schlechtgerüchen besonders in Verbindung mit Feuchtigkeit, beispielsweise bei gewaschenen oder aus anderen Gründen angefeuchteten Textilien oder feuchten harten Oberflächen. Besonders bevorzugt ist das erfindungsgemäße Mittel deshalb ein flüssiges Wasch- oder Reinigungsmittel, ein festes Wasch- oder Reinigungsmittel, ein Weichspüler, ein Hygienespüler oder ein Trocknertuch.

Je nach Art des erfindungsgemäßen Mittels, enthält das Mittel 0,01 bis 40 Gew.-%, bevorzugt 0,1 bis 10 Gew.-% der erfindungsgemäßen Duftstoffmischung.

Es konnte zudem gezeigt werden, dass sich der Schlechtgeruch am effektivsten verringern lässt und die Parfümierung in den Vordergrund der Duftwahrnehmung tritt, wenn das erfindungsgemäße Mittel einen pH-Wert im Bereich von 5 bis 9 aufweist. Bevorzugt liegt der pH-Wert erfindungsgemäßer Mittel deshalb im Bereich von 2,5 bis 10,5 und insbesondere im Bereich von 5 bis 9.

In den erfindungsgemäßen Mitteln sind mit Vorzug mehr als 5 Gew.-%, bevorzugt mehr als 10 Gew.-% Tensid und insbesondere mehr als 15 Gew.-% Tensid - bezogen auf das Mittel - enthalten. Ausführungsformen mit besonders hohem Tensidgehalt können mehr als 20 Gew.-% Tensid, mehr als 25 Gew.-% Tensid, mehr als 30 Gew.-% Tensid oder sogar mehr als 35 Gew.-% Tensid enthalten. Es ist jedoch bevorzugt, dass der Tensidgehalt des Mittels unterhalb von 70 Gew.-%, mit Vorzug unterhalb von 60 Gew.-% und insbesondere unterhalb von 50 Gew.-% liegt.

Als anionisches Tensid können vorzugsweise Sulfonate und/oder Sulfate eingesetzt werden.

Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch C₁₂₋₁₈-Alkansulfonate und die Ester von α-Sulfofettsäuren (Estersulfonate), zum Beispiel die α-sulfonierten Methylester der hydrierten Kokos-, Palmkern- oder Talgfettsäuren.

Als Alk(en)ylsulfate werden die Alkali- und insbesondere die Natriumsalze der Schwefelsäurehalbester der C₁₂₋C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete anionische Tenside.

Auch Fettalkoholethersulfate, wie die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Weitere geeignete anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium-, Magnesium- oder Ammoniumsalze vorliegen. Geeignete Ammoniumsalze als Gegenionen für die anionischen Tenside sind die protonierten Formen von Cholin, Triethylamin, Monoethanolamin, Triethanolamin oder Methylethylamin.

Geeignete nichtionische Tenside umfassen alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus.

Als nichtionisches Tensid werden vorzugsweise alkoxylierte, vorteilhafterweise ethoxylierte, insbesondere primäre Alkohole mit vorzugsweise 8 bis 18 C-Atomen und durchschnittlich 4 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 5 bis 8 EO pro Mol Alkohol bevorzugt. Zu den bevorzugten ethoxylierten Alkoholen gehören beispielsweise C₁₂₋₁₄-Alkohole mit 4 EO oder 7 EO, C₉₋₁₁-Alkohol mit 7 EO, C₁₃₋₁₅-Alkohole mit 5 EO, 7 EO oder 8 EO, C₁₂₋₁₈-Alkohole mit 5 EO oder 7 EO und Mischungen aus diesen. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆₋₁₈-Fettalkohol mit 7 EO und 2-Propylheptanol mit 7 EO. Insbesondere bevorzugt enthält das erfindungsgemäße Mittel einen C₁₂₋₁₈-Fettalkohol mit 7 EO oder einen C₁₃₋₁₅-Oxoalkohol mit 7 EO oder 8 EO als nichtionisches Tensid.

Ein Vorteil der vorliegenden Erfindung ist, dass der Einsatz keimtötender kationischer Tenside reduziert werden oder sogar ganz auf diese Tenside verzichtet werden kann. Bevorzugt enthalten erfindungsgemäße Mittel dementsprechend weniger als 5 Gew.-%, vorzugsweise weniger als 1 Gew.-% und insbesondere kein kationisches Tensid.

Gegenstand der vorliegenden Anmeldung ist die Verwendung einer Duftstoffmischung, welche Hexylsalicylat und mindestens ein weiteres Salicylat umfasst, wobei das weitere Salicylat kein 3,7,11-Trimethyl-2,6,10-dodecatrien-1-yl-2-hydroxybenzoat ist, sowie eines Mittels, welches eine solche Duftstoffmischung enthält, zur Verminderung von Schlechtgerüchen.

Hierbei ist die erfindungsgemäße Duftstoffmischung bevorzugt in flüssigen Wasch- oder Reinigungsmitteln, festen Wasch- oder Reinigungsmitteln, Weichspülern, Hygienespülern oder Trocknertüchern enthalten.

In einem weiteren Aspekt betrifft die vorliegende Erfindung ein Verfahren zur Herstellung eines Schlechtgerüche vermindernden Wasch- oder Reinigungsmittels, Hygienespülers oder Trocknertuchs, in welchem eine Duftstoffmischung, welche Hexylsalicylat und mindestens ein weiteres Salicylat umfasst, wobei das weitere Salicylat kein 3,7,11-Trimethyl-2,6,10-dodecatrien-1-yl-2-hydroxybenzoat ist, mit weiteren üblichen Inhaltsstoffen von Wasch- oder Reinigungsmitteln, Weichspülern, Hygienespülern oder Trocknertüchern vermischt wird.

Handelt es sich bei dem erfindungsgemäßen Mittel um ein Trocknertuch, wird die erfindungsgemäße Duftstoffmischung oder vorzugsweise auf das Gewebe des Trocknertuches aufgesprüht. Ist das erfindungsgemäße Mittel flüssig, wird die erfindungsgemäße Duftstoffmischung mit Vorzug mit anderen flüssigen Mittel-Bestandteilen verrührt.

Die Erfindung betrifft zudem ein Verfahren zur Herstellung eines Schlechtgerüche vermindernden festen Wasch- oder Reinigungsmittels, in welchem eine erfindungsgemäße Duftstoffmischung auf einem pulverförmigen Träger aufgebracht und das resultierende Produkt mit weiteren Inhaltsstoffen des Wasch- oder Reinigungsmittels vermischt wird.
Als pulverförmiger Träger wird mit Vorzug Silica, Natriumcarbonat, Natiumsulfat, Zeolith, Natiumsilikat, Aluminosilikat oder ein Gemisch umfassend mehrere der hier genannten Komponenten eingesetzt.

In einem anderen Aspekt betrifft die Erfindung ein Verfahren zur Herstellung eines Schlechtgerüche vermindernden festen Wasch- oder Reinigungsmittels, in welchem eine erfindungsgemäße Duftstoffmischung in nichtionisches Tensid eingemischt und das resultierende Produkt mit weiteren Inhaltsstoffen des Wasch- oder Reinigungsmittels vermischt wird. Bevorzugt wird das in diesem Verfahren hergestellte Gemisch umfassend die erfindungsgemäße Duftstoffmischung und nichtionisches Tensid in einem der Post-Additions-Schritte aufgebracht.

Zusätzlich zur erfindungsgemäßen Duftstoffmischung und dem enthaltenen Tensid kann das erfindungsgemäße Mittel weitere Inhaltsstoffe enthalten.

Flüssige Wasch- oder Reinigungsmittel können beispielsweise weitere Inhaltsstoffe enthalten, welche die anwendungstechnischen und/oder ästhetischen Eigenschaften des Wasch- oder Reinigungsmittels weiter verbessern. Im Rahmen der vorliegenden Erfindung enthält ein erfindungsgemäßes Wasch- oder Reinigungsmittel vorzugsweise zusätzlich einen oder mehrere Stoffe aus der Gruppe der Gerüststoffe, Bleichmittel, Bleichkatalysatoren, Bleichaktivatoren, Enzyme, Elektrolyte, pH-Stellmittel, Parfüme, Parfümträger, Fluoreszenzmittel, Farbstoffe, Hydrotope, Schauminhibitoren, Silikonöle, Einlaufverhinderer, Knitterschutzmittel, Farbübertragungsinhibitoren, Antioxidantien, Konservierungsmittel, Korrosionsinhibitoren, Antistatika, Bittermittel, Bügelhilfsmittel, Phobier- und Imprägniermittel, Haut-pflegende Wirkstoffe, Quell- und Schiebefestmittel, weichmachenden Komponenten sowie UV-Absorber.

### Referenzbeispiel:

**Tabelle 1: Duftstoffmischungen**

| | Hexylsalicylat | *i*-Amylsalicylat | Cyclohexylsalicylat |
|---|---|---|---|
| D1 | 125 | 25 | 10 |
| D2 | 150 | - | 10 |
| V1 | 160 | - | - |

### [angegeben sind Gewichtsverhältnisse]

Die erfindungsgemäßen Duftstoffmischungen D1 und D2 enthalten neben Hexylsalicylat mindestens ein weiteres Salicylat. Im Vergleichsbeispiel V1 wird dem Hexylsalicylat kein weiteres Salicylat zugemischt.

Die Duftstoffmischungen aus der Tabelle 1 werden in einer Menge von 1,5 Gew.-% - bezogen auf 100 Gew.-% Mittel plus Duftstoffmischung - in folgendes flüssige Waschmittel eingemischt.

**Tabelle 2: Flüssigwaschmittel**

| | Mittel 1 | Mittel 2 | Mittel 3 |
|---|---|---|---|
| | umfassend D1 | umfassend D2 | umfassend V1 |
| C₁₂₋₁₈ROH (7 EO) | 12,0 | 12,0 | 12,0 |
| C₁₂₋₁₄-Dimethylaminoxid | 2,0 | 2,0 | 2,0 |
| C₁₂₋₁₈-Fettsäure | 2,5 | 2,5 | 2,5 |
| H₂O₂ (35 %ig) | 0,2 | 0,2 | 0,2 |
| Citronensäure·H₂O | 0,1 | 0,1 | 0,1 |
| Natronlauge (50%-ig) zur Einstellung des pH-Wertes auf | 3 | 3 | 8,2 |
| D1, D2 bzw. V1 | 1,5 | 1,5 | 1,5 |
| Wasser | ad 100 | ad 100 | ad 100 |

### [alle Mengen sind in Gew.-% Aktivstoff, bezogen auf die gesamte Zusammensetzung, angegeben]

In einer Miele Novotronic W526 wurden je zwei Polyester- und Baumwollgewebe bei einem Wasserlevel von 7,6 L und 20 °C und einer Dosierung von 80 g mit den Mitteln 1 bis 3 behandelt. Zu jedem Waschversuch wurde je 100 g alkalischer und sauerer Kunstschweiß zudosiert.

Die Testgewebe wurden mit sauberem Wasser klargespült und 24 Stunden in der geschlossenen Waschmaschine gelagert.

Anschließend wurden sie unabhängig von drei parfümistisch geschulten Personen abgerochen, wobei jeweils der Parfüm- und der Schlechtgeruch bestimmt wurden. Hierbei wurde das Verhältnis von Schlechtgeruch zu Parfümgeruch bestimmt. Die Intensitäten der Gerüche wurden mittels einer Skala von 0 bis 10 (0 nicht wahrnehmbar, 10 extremer Geruchseindruck) bewertet.

**Tabelle 3: Ergebnisse Parfüm- und Schlechtgeruchbestimmung**

| | Mittel 1 umfassend D1 | Mittel 2 umfassend D2 | Mittel 3 umfassend V1 |
|---|---|---|---|
| Schlechtgeruch | 2 | 3 | 4 |

| | | | |
|---|---|---|---|
| (Mittelwert aus 3 unabhängigen Einzelbestimmungen; 0 = kein Schlechtgeruch; 6 extrem starker Schlechtgeruch) | | | |

Für den Einsatz der erfindungsgemäßen Mittel 1 und 2 konnte eine deutliche Verminderung des Schlechtgeruchs der Textilien durch geschulte Testpersonen festgestellt werden. Im Fall des Einsatzes des nicht erfindungsgemäßen Mittels 3 war nur hingegen ein starker Schlechtgeruch wahrnehmbar.

## Patentansprüche

1. Duftstoffmischung enthaltend Hexylsalicylat und zusätzlich mindestens ein weiteres Salicylat, wobei das weitere Salicylat kein 3,7,11-Trimethyl-2,6,10-dodecatrien-1-yl-2-hydroxybenzoat ist und wobei die Mischung zusätzlich zum Hexylsalicylat und zum mindestens einen weiteren Salicylat ein drittes Salicylat, das 3,7,11-Trimethyl-2,6,10-dodecatrien-1-yl-2-hydroxybenzoat ist, enthält.

2. Mischung gemäß Anspruch 1, **dadurch gekennzeichnet, dass** das weitere Salicylat in der Mischung ausgewählt ist aus der Gruppe, die gebildet wird durch Cyclohexylsalicylat, Hexenylsalicylat, Methylsalicylat, Benzylsalicylat, Methylsalicylat, n-Amylsalicylat, *i*-Amylsalicylat, 2,2,4-(2,4,4-) Trimethylcyclopentylsalicylat als Isomerengemisch, Ethylhexylsalicylat, Cyclooctylsalicylat, 4-Isopropylcyclohexylsalicylat, Cyclohexylmethylsalicylat, Cycloheptylsalicylat, Cyclooctyl-salicylat und Cyclooct-4-enyl-salicylat.

3. Mischung gemäß einem der Ansprüche 1 oder 2, **dadurch gekennzeichnet, dass** diese mindestens 2 Gew.-% Hexylsalicylat - bezogen auf die Mischung - enthält.

4. Mischung gemäß einem der Anspruche 1 bis 3, **dadurch gekennzeichnet, dass** die Parfümhaftung der restlichen Parfüminhaltsstoffe durch die Mischung der Salicylate verbessert wird.

5. Mischung gemäß einem der Anspruche 1 bis 4, **dadurch gekennzeichnet, dass** diese 5 bis 100 Gew.-% Salicylate, insbesondere 10 bis 30 Gew.-% Salicylate - bezogen auf die Mischung - umfasst.

6. Mittel umfassend mindestens ein Tensid aus der Gruppe der nichtionischen oder anionischen Tenside und eine Mischung gemäß einem der Ansprüche 1 bis 5, wobei das Mittel mehr als 5 Gew.-%, insbesondere mehr als 10 Gew.-% Tensid enthält.

7. Mittel gemäß Anspruch 6, **dadurch gekennzeichnet, dass** das Mittel ein festes Wasch- oder Reinigungsmittel, ein flüssiges Wasch- oder Reinigungsmittel, ein Hygienespüler, ein Aftershaveprodukt, ein Seifenprodukt, ein Duschgel, eine Duschcreme, ein Badesalz, ein Badeschaum, ein Haarpflegeprodukt, ein Shampoo, ein Körperpflegeprodukt, ein Deodorant, eine kosmetische Zubereitung, ein Trocknertuch, ein Bügelwasser oder ein Wischtuch ist.

8. Mittel gemäß einem der Ansprüche 6 oder 7, **dadurch gekennzeichnet, dass** das Mittel 0,01 bis 40 Gew.-%, bevorzugt 0,1 bis 10 Gew.-% einer Mischung gemäß einem der Ansprüche 1 bis 5 enthält.

9. Mittel gemäß einem der Ansprüche 6 bis 8, **dadurch gekennzeichnet, dass** das Mittel einen pH-Wert im Bereich von 2,5 bis 10,5 und insbesondere von 5 bis 9 aufweist.

10. Mittel gemäß einem der Ansprüche 7 bis 9, **dadurch gekennzeichnet, dass** das Mittel weniger als 5 Gew.-%, vorzugsweise weniger als 1 Gew.-% und insbesondere kein kationisches Tensid enthält.

11. Verwendung einer Mischung gemäß einem der Ansprüche 1 bis 5 oder eines Mittels umfassend eine Mischung gemäß einem der Ansprüche 1 bis 5 zur Verminderung von Schlechtgerüchen.

12. Verfahren zur Herstellung eines Schlechtgerüche vermindernden Wasch- oder Reinigungsmittels, Weichspülers, Hygienespülerns oder Trocknertuchs, in welchem eine Mischung gemäß einem der Ansprüche 1 bis 5 mit weiteren üblichen Inhaltsstoffen von Wasch- oder Reinigungsmitteln, Weichspülern, Hygienespülern oder Trocknertüchern vermischt wird.

## Claims

1. A fragrance mixture containing hexyl salicylate and additionally at least one further salicylate, wherein the further salicylate is not 3,7,11-trimethyl-2,6,10-dodecatrien-1-yl-2-hydroxybenzoate, and wherein the mixture contains, in addition to hexyl salicylate and the at least one further salicylate, a third salicylate, which is 3,7,11-trimethyl-2,6,10-dodecatrien-1-yl-2-hydroxybenzoate.

2. The mixture according to claim 1, **characterized in that** the further salicylate in the mixture is selected from the group consisting of cyclohexyl salicylate, hexenyl salicylate, methyl salicylate, benzyl salicylate, methyl salicylate, n-amyl salicylate, i-amyl salicylate, 2,2,4-(2,4,4-)trimethylcyclopentyl salicylate as an isomeric mixture, ethylhexyl salicylate, cyclooctyl salicylate, 4-isopropylcyclohexyl salicylate, cyclohexylmethyl salicylate, cycloheptyl salicylate, cyclooctyl salicylate, and cyclooct-4-enyl salicylate.

3. The mixture according to one of claims 1 or 2, **characterized in that** it contains at least 2 wt.% of hexyl salicylate, based on the mixture.

4. The mixture according to one of claims 1 to 3, **characterized in that** the perfume adhesion of the remaining perfume ingredients is improved by the mixture of the salicylates.

5. The mixture according to one of claims 1 to 4, **characterized in that** it comprises from 5 to 100 wt.% of salicylates, in particular from 10 to 30 wt.% of salicylates, based on the mixture.

6. An agent comprising at least one surfactant from the group of non-ionic or anionic surfactants and a mixture according to one of claims 1 to 5, wherein the agent contains more than 5 wt.%, in particular more than 10 wt.%, of surfactant.

7. The agent according to claim 6, **characterized in that** the agent is a solid washing or cleaning agent, a liquid washing or cleaning agent, a hygiene rinser, an aftershave product, a soap product, a shower gel, a shower cream, a bath salt, a bath foam, a hair care product, a shampoo, a body care product, a deodorant, a cosmetic preparation, a dryer sheet, ironing water or a wipe cloth.

8. The agent according to one of claims 6 or 7, **characterized in that** the agent contains from 0.01 to 40 wt.%, preferably from 0.1 to 10 wt.%, of a mixture according to one of claims 1 to 5.

9. The agent according to one of claims 6 to 8, **characterized in that** the agent has a pH in the range of from 2.5 to 10.5, and in particular from 5 to 9.

10. The agent according to one of claims 7 to 9, **characterized in that** the agent contains less than 5 wt.% of a cationic surfactant, preferably less than 1 wt.% of a cationic surfactant, and in particular no cationic surfactant.

11. The use of a mixture according to one of claims 1 to 5 or an agent comprising a mixture according to one of claims 1 to 5 for reducing unpleasant odors.

12. A method for producing a washing or cleaning agent, fabric softener, hygiene rinser or dryer sheet that reduces unpleasant odors, in which a mixture according to one of claims 1 to 5 is mixed with further conventional ingredients of washing or cleaning agents, fabric softeners, hygiene rinsers, or dryer sheets.

## Revendications

1. Mélange de substances odorantes contenant du salicylate d'hexyle et en plus au moins un autre salicylate, l'autre salicylate n'étant pas du 3,7,11-triméthyl-2,6,10-dodécatrièn-1-yl-2-hydroxybenzoate et le mélange contenant en plus du salicylate d'hexyle et de l'au moins un autre salicylate un troisième salicylate qui est le 3,7,11-triméthyl-2,6,10-dodécatrièn-1-yl-2-hydroxybenzoate.

2. Mélange selon la revendication 1, **caractérisé en ce que** l'autre salicylate du mélange est sélectionné dans le groupe formé par le salicylate de cyclohexyle, le salicylate d'hexényle, le salicylate de méthyle, le salicylate de benzyle, le salicylate de méthyle, le salicylate de n-amyle, le salicylate de *i*-amyle, le salicylate de 2,2,4-(2,4,4)-triméthylcyclopentyle sous la forme d'un mélange d'isomères, le salicylate d'éthylhexyle, le salicylate de cyclooctyle, le salicylate de 4-isopropylcyclohexyle, le salicylate de cyclohexylméthyle, le salicylate de cycloheptyle, le salicylate de cyclooctyle et le salicylate de cyclooct-4-ényle.

3. Mélange selon l'une des revendications 1 ou 2, **caractérisé en ce qu'**il contient au moins 2 % en poids de salicylate d'hexyle, par rapport au mélange.

4. Mélange selon l'une des revendications 1 à 3, **caractérisé en ce que** l'adhérence des substances odorantes restantes est améliorée par le mélange des salicylates.

5. Mélange selon l'une des revendications 1 à 4, **caractérisé en ce qu'**il contient de 5 à 100 % en poids de salicylate, en particulier de 10 à 30 % en poids de salicylate, par rapport au mélange.

6. Agent comprenant au moins un tensioactif du groupe des tensioactifs non ioniques ou anioniques et un mélange selon l'une des revendications 1 à 5, l'agent contenant plus de 5 % en poids, en particulier plus de 10 % en poids, de tensioactif.

7. Agent selon la revendication 6, **caractérisé en ce que** l'agent est un agent de nettoyage ou de lavage solide, un agent de nettoyage ou de lavage liquide, un produit de rinçage désinfectant, un produit après-rasage, un produit à base de savon, un gel douche, une crème douche, un sel de bain, une mousse de bain, un produit de soin capillaire, un shampooing, un produit de soin corporel, un déodorant, une préparation cosmétique, une lingette, une eau de repassage ou un chiffon.

8. Agent selon l'une des revendications 6 ou 7, **caractérisé en ce que** l'agent contient de 0,01 à 40 % en poids, de préférence de 0,1 à 10 % en poids d'un mélange selon l'une des revendications 1 à 5.

9. Agent selon l'une des revendications 6 à 8, **caractérisé en ce que** l'agent a un pH dans la gamme de 2,5 à 10,5, et en particulier de 5 à 9.

10. Agent selon l'une des revendications 7 à 9, **caractérisé en ce que** l'agent contient moins de 5 % en poids, de préférence moins de 1 % en poids de tensioactif cationique et en particulier ne contient aucun tensioactif cationique.

11. Utilisation d'un mélange selon l'une des revendications 1 à 5 ou d'un agent comprenant un mélange selon l'une des revendications 1 à 5, pour la réduction des mauvaises odeurs.

12. Procédé de production d'un agent de nettoyage ou de lavage, un agent adoucissant, un agent de rinçage hygiénique ou une serviette réduisant les mauvaises odeurs, dans lequel un mélange selon l'une des revendications 1 à 5 est mélangé à d'autres ingrédients usuels des agents de nettoyage ou de lavage, des agents assouplissants, des agents de rinçage désinfectants ou des lingettes.
